(19) Europäisches Patentamt

European Patent Office

Office européen des brevets

(11) **EP 1 034 753 B1**

(12) **EUROPÄISCHE PATENTSCHRIFT**

(45) Veröffentlichungstag und Bekanntmachung des Hinweises auf die Patenterteilung:
**16.02.2005 Patentblatt 2005/07**

(51) Int Cl.7: **A61F 2/24**

(21) Anmeldenummer: **00102099.9**

(22) Anmeldetag: **03.02.2000**

(54) **Anuloplastiering**

Annuloplasty ring

Anneau pour annuloplastie

(84) Benannte Vertragsstaaten:
**AT BE CH DE DK ES FI FR GB GR IE IT LI LU MC NL PT SE**

(30) Priorität: **09.03.1999 DE 19910233**

(43) Veröffentlichungstag der Anmeldung:
**13.09.2000 Patentblatt 2000/37**

(73) Patentinhaber: **Maquet Cardiopulmonary AG
72145 Hirrlingen (DE)**

(72) Erfinder:
- **Prof. Dr. med. Maazouzi Wajih
Villa 119 Rabat (MA)**
- **Dr. Ing. Ralf Kaufmann
72414 Rangendingen (DE)**

(74) Vertreter: **Möbus, Daniela, Dr.-Ing. et al
Patentanwältin Dr. Möbus
Kaiserstrasse 85
72764 Reutlingen (DE)**

(56) Entgegenhaltungen:
**EP-A- 0 595 791          WO-A-95/03757**

EP 1 034 753 B1

**Beschreibung**

Die Erfindung betrifft eine Annuloplastieprothese für mitrale und trikuspidale Herzklappen.

Annuloplastieprothesen werden bei Herzklappeninsuffizienzen zur Umgehung der Implantation einer künstlichen Herzklappe eingesetzt. Der Einsatz einer Annuloplastieprothese wird dabei gegebenenfalls mit einer Klappenrekonstruktion, einer Verkürzung oder Transposition der Papillarsehnenfäden oder einer Verkürzung der Papillarmuskeln kombiniert.

Der atrioventrikuläre Annulus ist eine dynamische Struktur, die während des Herzzyklus Änderungen in Größe und Form unterliegt. Während der Systole verringert sich insbesondere der mittlere Annulusdurchmesser der Mitralklappe durch eine posteriore Annularkontraktion, während die anteriore Annularlänge nahezu unverändert bleibt. Falls eine systolische anteriore annulare Bewegung (systolic anterior motion, SAM) vorliegt und darüber hinaus ein zu großes posteriores Klappensegel existiert, kann es zu Behinderungen des linksventrikulären Auswurfs (left ventricular outflow tract obstruction, LVOTO) kommen.

Zu den anuloplastisch therapierbaren Insuffizienzen gehört vor allem der nicht stenotisch bedingte Verlust an Schlagvolumen, hervorgerufen durch überhöhten systolischen Rückfluss des Blutes durch die Mitral- bzw. Trikuspidalklappe in den entsprechenden Vorhof. Für diese Insuffizienzen können chronische Ursachen wie Klappenprolaps, links- und rechtsventrikuläre Dilatation, Überdehnung der Pulmonalarterie oder linksventrikuläre Klappenerkrankungen, die die Funktion der Trikuspidalklappe beeinträchtigen, vorliegen. Diese Symptome werden beispielsweise durch rheumatisches Fieber, koronare Krankheit, kalzifizierten Anulus, Marfan-Syndrom, Fehlfunktionen der Papillarmuskeln und Lupus hervorgerufen. Für diese Insuffizienzen können jedoch auch akute Ursachen vorliegen wie Klappenprolaps durch Ruptur der Papillarsehnenfäden (Myxom, Endokarditis, Trauma) und Ruptur des Papillarmuskels (Infarkt, Trauma) sowie die Perforation eines Klappensegels (Endokarditis).

Ziel der Mitral- bzw. Trikuspidalklappenrekonstruktion durch Implantation einer Anuloplastieprothese ist letztlich die Schaffung einer möglichst breiten Koaptationsfläche der Klappensegel und eine Gewebeentlastung während der Systole sowie eine gute Hämodynamik während der Diastole. Dies beinhaltet die Korrektur der Dilatation und/oder Deformation des Anulus, die selektive Reduktion deformierter Bereiche der Klappensegel sowie die Prävention von wiederkehrenden Dilatationen und Deformationen. Bisher werden starre oder flexible Mitralklappenrekonstruktionsringe (Carpentierbzw. Duranringe) verwendet, die eine Reduzierung des Rings der Mitralklappe bewirken und dadurch zu einer erhöhten Schließfähigkeit führen. Die starren Anuloplastieringe, wie sie beispielsweise in der US-A 3 656 185 offenbart sind, zeichnen sich durch eine hohe Formstabilität aus. Die flexiblen Anuloplastieringe weisen dagegen den Vorteil einer zyklischen Verformbarkeit auf. Sie zeigen eine hohe strukturelle Torsionsfähigkeit und eine äußerst geringe Umfangsdehnbarkeit. Dies gewährleistet eine deutliche Gewebeentlastung der atrioventrikulären Annulie. Computersimulationen und praktische Messungen mit einem 3-D-Echokardiografieverfahren haben die Vorteile dieser flexiblen Ringe gegenüber den schon länger bekannten starren Annuloplastieringen deutlich nachgewiesen. Während der Systole verringert sich hier die druckbeaufschlagte gesamte Klappenfläche um ca. 25 Prozent (Kunzelmann K.S. et al: Flexible versus rigid annuloplasty for mitral valve annular dilatation: a finite element model. J. Heart Valve Dis. 1998; 7, 1: 108-116 und Yamaura Y et al.: Three-dimensional echocardiographic evaluation of configuration and dynamics of the mitral annulus in patients fitted with an annuloplasty ring. J. Heart Valve Dis. 1997; 6,1: 43-47).

Für die diastolische Ventrikelfüllung steht umgekehrt eine größere atrioventrikuläre Öffnungsfläche und somit ein geringerer Einströmwiderstand als bei einem völlig starren Annuloplastiering zur Verfügung. Auch das SAM-Syndrom tritt nach der Implantation von flexiblen Annuloplastiken wesentlich weniger häufig auf als nach der Implantation von starren Ringen. Nachteilig an den bis auf die Umfangslänge völlig flexiblen Annuloplastieringen ist jedoch, dass es nach der Implantation, die an einem entlasteten und stillstehenden Annulus durchgeführt werden muss, zu periodischen Faltenbewegungen und Ausbeulungen der Klappensegel, vor allem im Bereich der Kommissuren, kommen kann.

Die bekannte Annuloplastieprothese nach WO 95/03757 ist aus einem geschlossenen Ring gebildet, der einen Kern mit einer sektoralen unterschiedlichen Biegeflexibilität in der Ringebene aufweist. Der Kern kann als geschlossener Ring oder als Abschnitt eines Rings ausgebildet sein. Die bekannte Annuloplastieprothese weist in ihrer Anwendung als mitrale und trikuspitale Annuloplastik unterschiedliche Ringformen auf.

Der vorliegenden Erfindung liegt die Aufgabe zugrunde, eine Annuloplastieprothese zu schaffen, die die Vorteile flexibler Annuloplastieringe aufzeigt, ohne mit deren Nachteilen behaftet zu sein.

Die Aufgabe wird mit einer Annuloplastieprothese für mitrale und trikuspidale Herzklappen gelöst, die die Merkmale des Patentanspruchs 1 aufweist.

Eine Annuloplastieprothese mit Bereichen unterschiedlicher Flexibilität ist zwar bereits aus der WO 97/16135 bekannt, doch wird hier die Flexibilität durch mehr oder weniger weit geöffnete Ringformen erreicht. Die Annuloplastieprothesen gemäß der Erfindung erhalten die gewünschten Flexibilitätseigenschaften durch die Aufteilung des offenen Rings in Sektoren. So kann der Kern eine hohe Biegeflexibilität im mittleren Bereich - dem Bereich des posterioren mitralen Klappensegels - und eine hohe Biegesteifigkeit im Bereich der Klappenkommissuren aufweisen. Dies kann beispielsweise dadurch erzielt werden, dass der Kern über seine Länge Sektoren mit unterschiedlichen Profilen aufweist. Dabei können die unterschiedlichen Profile stetig oder unstetig ineinander übergehen.

Durch die mechanischen Verformungseigenschaften der sektoriellen Profilierung des erfindungsgemäßen Annuloplastieringkerns werden in einfacher Weise die zyklische Verformbarkeit bekannter flexibler Annuloplastieringe und die gute Formstabilität bekannter starrer Annuplastieringe kombiniert.

Besondere Vorteile ergeben sich, wenn der Kern aus einem einheitlichen Werkstoff hergestellt ist. Die Bruchsicherheit des Kerns lässt sich dann sehr viel leichter kontrollieren als bei gefügten Kernen. Als Materialien für den Kern kommen Metalle oder eine Titanlegierung in Frage. Dabei kann der Kern vorzugsweise aus einem Rund- oder Vielkantdraht gefertigt sein. Die unterschiedlichen Profile des Kerns ergeben sich aus einem kaltverformten Draht. Durch die Kaltverformung des Drahtes erhält dieser eine höhere Zähigkeit als bei einer Warmumformung.

Der Draht kann mindestens bereichsweise einen im Wesentlichen rechteckigen Querschnitt aufweisen, wobei die Höhe und Breite des Querschnitts über die Länge des Drahtes variiert. Je nachdem, in welcher Richtung der Draht abgeplattet wird, verändert sich auch die Flexibilität gegen Krafteinwirkung in der Ringebene. Weist der Kern nur eine geringe Dicke in der radialen Richtung des Annuloplastieringes auf, so ist der gesamte Ring in diesem Bereich sehr leicht biegbar. Umgekehrt führt ein in der Ringebene abgeplatteter Kern zu einer hohen radialen Steifigkeit des Kerns, sodass sich der Annuloplastiering in diesen Bereichen nur wenig verbiegen lässt. Durch Veränderung des Verhältnisses der Profilhöhe zur Profilbreite können Änderungen des Flächenträgheitsmoments des Kerns von bis zu 40 Prozent gegenüber dem unmodifizierten Profil erreicht werden.

In einer bevorzugten Ausführungsform kann der Kern von einer biokompatiblen Ummantelung, beispielsweise einem Schlauch aus expandiertem Polytetrafluoroethylen (ePTFE) umgeben sein. Zwischen dem Kern und der Ummantelung kann eine Polsterschicht, beispielsweise aus einem Polyestergestrick, angeordnet sein.

Weitere Vorteile ergeben sich, wenn der Ring aus der Ringebene in Blutdurchflussrichtung in Richtung Vorhof konvex gewölbt ist.

Nachfolgend wird eine bevorzugte Ausführungsform eines erfindungsgemäßen Annuloplastierings anhand der Zeichnung näher erläutert.

In Einzelnen zeigen:

Fig. 1 eine Ansicht auf die Hauptebene eines offenen Anuloplastieringes;

Fig. 2 eine Ansicht von unten auf den Anuloplastiering aus Fig. 1;

Fig. 3 einen Schnitt entlang der Linie III-III durch den Anuloplastiering aus Fig. 1;

Fig. 4 einen Schnitt entlang der Linie IV-IV durch den Anuloplastiering nach Fig. 1;

Fig. 5 einen Schnitt entlang der Linie V-V durch den Anuloplastiering aus Fig. 1;

Fig. 6 eine Darstellung des Verlaufs des axialen Flächenträgheitsmoments $I_z$ in Abhängigkeit des Verhältnisses H/B;

Fig. 7 eine der Fig. 1 entsprechende Darstellung des Anuloplastieringes bei diastolischer Form der Herzklappe;

Fig. 8 eine der Fig. 7 entsprechende Darstellung des Anuloplastierings bei systolischer Form der Herzklappe.

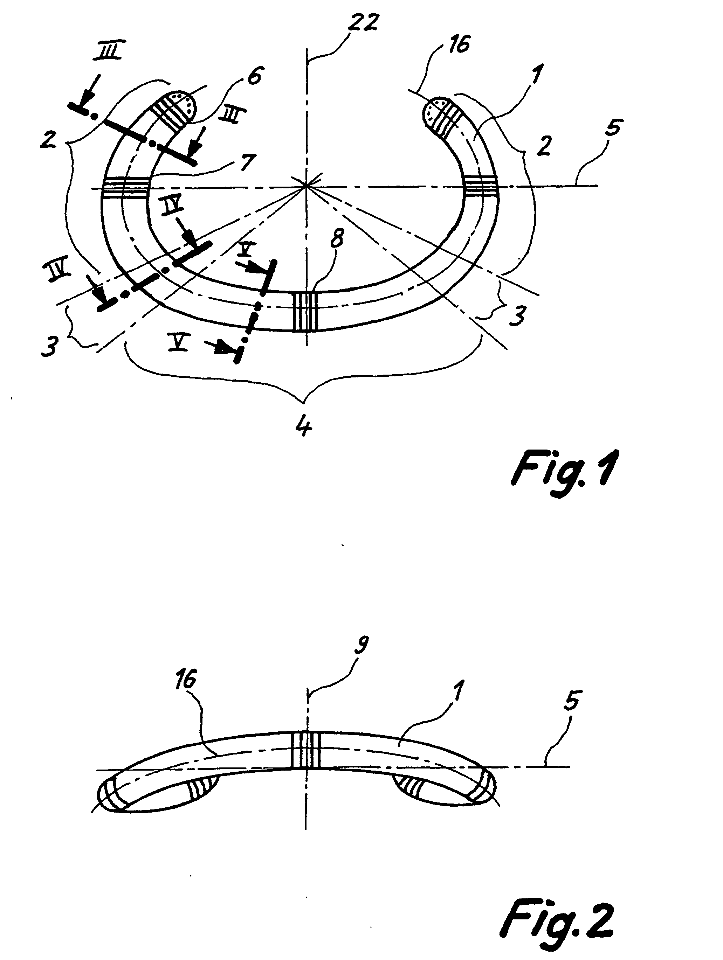

Der C-förmige Anuloplastiering 1 aus Fig. 1 weist eine Symmetrieachse 22 auf, die durch eine Achse 5, die durch den Bereich der größten Ringausdehnung verläuft, senkrecht geschnitten wird. Die Achse 5 und die Symmetrieachse 22 spannen die kinematische Hauptebene auf. Der Anuloplastiering 1 ist zur erleichterten Positionierung durch den Herzchirurgen an seinen Enden 6, an den Stellen größter Ringausdehnung 7 und an der Schnittstelle 8 der Symmetrieachse 22 mit Markierungen versehen. Diese Markierungen 6, 7, 8 können beispielsweise aus gefärbtem, geflochtenem Polyester-Nahtmaterial gefertigt sein. Die Linie 16 repräsentiert den Verlauf der neutralen Fasern des Annuloplastierings und unterliegt bei elastischen Formänderungen keinen Dehnungen. Sie ist die geometrische Richtgröße zur Berechnung der sektoriellen Flächenträgheitsmomente des Annuloplastierings 1.

In Fig. 2 wird verdeutlicht, dass der Annuloplastiering 1 aus der Hauptebene in Blutdurchflussrichtung nach vorn gewölbt, also konvex in Richtung Vorhof vorgeformt ist. Die Achse 9 in Fig. 2 ist orthogonal zu den Achsen 5 und 22 aus Fig. 1 und zeigt in die Z-Richtung, auf die die Flächenträgheitsmomente $I_z$ bezogen sind.

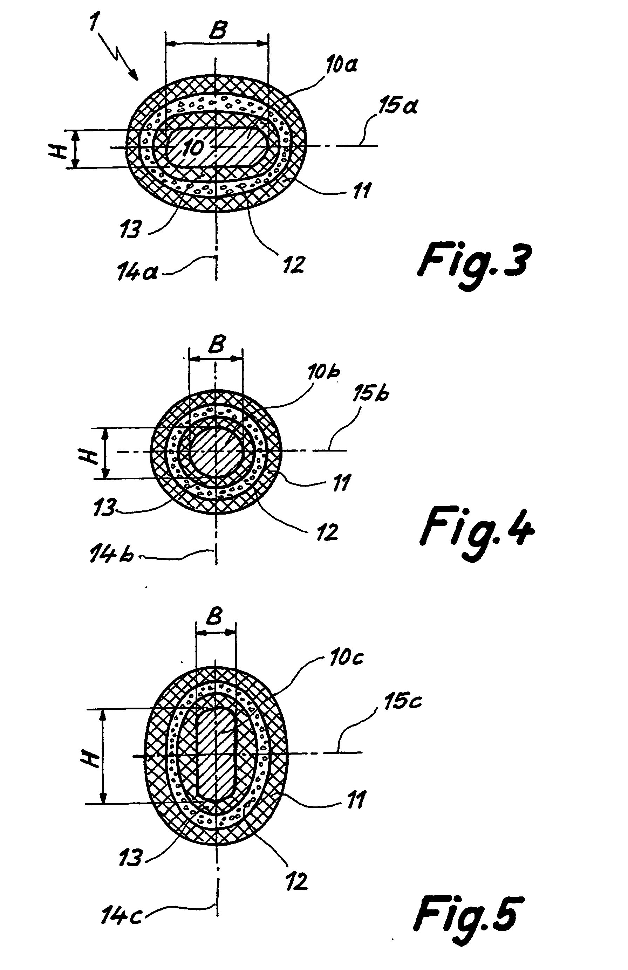

Der Ring 1 ist gemäß Fig. 1 in Sektoren 2, 3, 4 unterschiedlicher Biegeflexibilität in der kinematischen Hauptebene unterteilt. In den symmetrisch angeordneten Sektoren 2 besitzt der Ring 1 die größte Steifigkeit und im Bereich 4 die geringste Steifigkeit. Die Sektoren 3 sind Bereiche mittlerer Steifigkeit. Die unterschiedliche Steifigkeit wird durch einen im Inneren des offenen Rings 1 enthaltenen Kern 10 erreicht, der in den Bereichen 2, 3 und 4 ein unterschiedliches Profil aufweist. Dies ist in den Figuren 3 bis 5 gezeigt. Der Kern 10 wird dabei von von einem Runddraht gebildet, wobei die runde Querschnittsform gemäß Fig. 4 in den Bereichen 3 unverändert beibehalten wird.

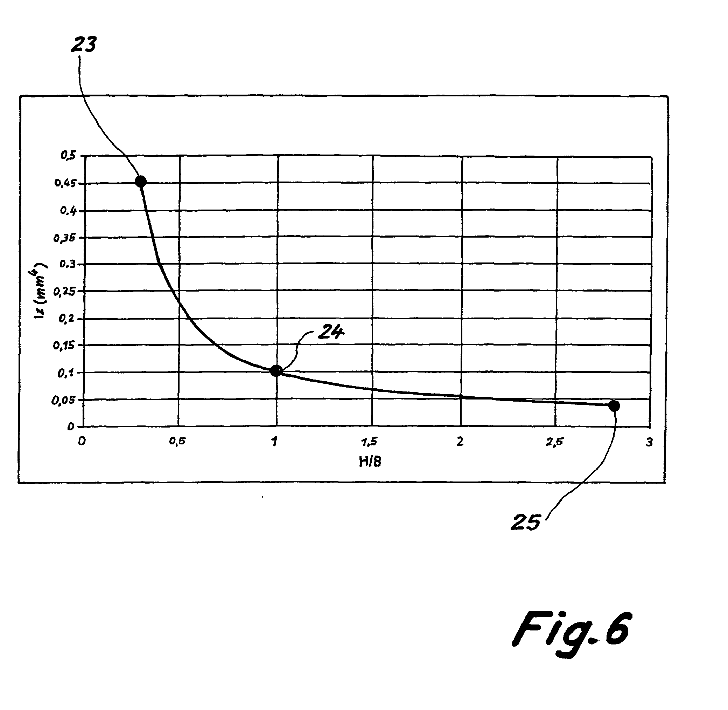

In Fig. 3 ist der gegen Kräfte in der kinematischen Hauptebene versteifte Sektor 2 des Annuloplastierings 1 (Fig. 1) im Schnitt dargestellt. Die Symmetrieachsen 14a und 15a sind parallel zur Achse 9 bzw. zur Hauptebene ausgerichtet. Sie sind stets orthogonal zur neutralen Faser 16 und schneiden sich in ihr. Durch das Verhältnis der Höhe H zur Breite B des Kernprofils 10a des Kerns 10 des Anuloplastieringes 1, das beispielsweise zwischen 0,35 und 1 liegen kann, ergibt sich ein relativ zum unmodifizierten, kreisförmigen Profil 10b (Fig. 4) hohes Flächenträgheitsmoment $I_z$, wie in Fig. 6 dargestellt ist. Damit ist auch eine größere Steifigkeit gegenüber äußeren Kräften, die innerhalb der Hauptebene wirken, gegeben. Bei der dargestellten Ausführungsform ist der sektoriell profilierte Kerndraht 10 von einem Schlauch 13 aus expandiertem Polytetrafluoroethylen umgeben. Als Polsterschicht 12 unter einer äußeren ePTFE-Ummantelung 11 kann ein Polyestergestrick eingesetzt werden.

In Fig. 4 ist der Anuloplastiering 1 im Sektor 3 mit unmodifiziertem Kernquerschnitt im Schnitt dargestellt. Die Symmetrieachsen 14b und 15b sind parallel zur Achse 9 (Fig. 2) bzw. zur Hauptebene (Fig. 1) ausgerichtet. Sie verlaufen auch orthogonal zur neutralen Faser 16 und schneiden sich in ihr. Durch das Verhältnis der Höhe H zur Breite B des Kernprofils 10b von 1 ergibt sich ein kleineres Flächenträgheitsmoment $I_z$ (Fig. 6) als im Sektor 2 und somit eine geringere Steifigkeit gegenüber äußeren Kräften, die innerhalb der Hauptebene wirken.

In Fig. 5 ist der Anuloplastiering 1 im Sektor 4 (Fig. 1) hoher Flexibilität im Schnitt dargestellt. Die Symmetrieachsen 14c und 15c sind wieder parallel zur Achse 9 (Fig. 2) und zur Hauptebene ausgerichtet. Sie verlaufen ebenfalls orthogonal zur neutralen Faser 16 und schneiden sich in ihr. Das Kernprofil 10c weist hier ein großes Verhältnis von Höhe H zur Breite B auf, beispielsweise im Bereich von 1 bis 2,86, wodurch sich im Vergleich zum unmodifizierten Profil 10b aus Fig. 4 ein niedrigeres Flächenträgheitsmoment ($I_z$) und somit eine geringere Steifigkeit gegenüber äußeren Kräften, die innerhalb der Hauptebene wirken, ergibt.

In Fig. 6 ist der interpolierte Verlauf des axialen Flächenträgheitsmomentes ($I_z$) in Abhängigkeit vom Höhen-Breiten-Verhältnis H/B am Ausführungsbeispiel eines abgeplatteten Rundkerndrahtes mit 1,2 mm Durchmesser dargestellt. Die Interpolation basiert auf drei gemessenen, durch Kaltverformung eines Runddrahtes herstellbaren H/B-Verhältnissen (siehe Punkte 23, 24, 25 im Diagramm aus Fig. 6).

Für den unmodifizierten Runddraht in Sektor 3 (Figuren 1 und 4) ist H = B = Kerndrahtdurchmesser und $I_z$ mit:

$$I_{zKreis} = 0,049 \cdot B^4$$

berechenbar (Punkt 24).

Für den sektoriell versteiften Kerndraht in Sektor 2 (Figuren 1 und 3) ist ein minimales Verhältnis H/B = 0,35 herstellbar, sodass $I_z$ in guter Näherung mit

$$I_{zRechteck} = 0,083 \cdot H \cdot B^3$$

angegeben werden kann (Punkt 23).

Für den sektoriell flexibilisierten Kerndraht in Sektor 4 (Figuren 1 und 5) ist ein maximales Verhältnis H/B = 2,86 herstellbar, sodass $I_z$ (Punkt 25) ebenfalls in guter Näherung wie Punkt 23 berechnet werden kann.

Flächenträgheitsmomente $I_z$ mit geometrischen Querschnittsübergängen vom Runddraht zum kaltverformten Vierkantdraht lassen sich für $0,35 \leq H/B \leq 2,86$ und einen vorgegebenen Drahtdurchmesser D von 1,2 mm des unmodifizierten Materials mit der allgemeinen Interpolationsgleichung:

$$I_z = D \cdot (- 0,214 + 0,225 \cdot e^{0,287 \cdot B/H}) \text{ mm}^4$$

berechnen (Fig. 6). Bei anderen Drahtdurchmessern müssen die Parameter der angesetzten Exponentialfunktion durch neue Stützpunkte (23, 24, 25) neu berechnet werden.

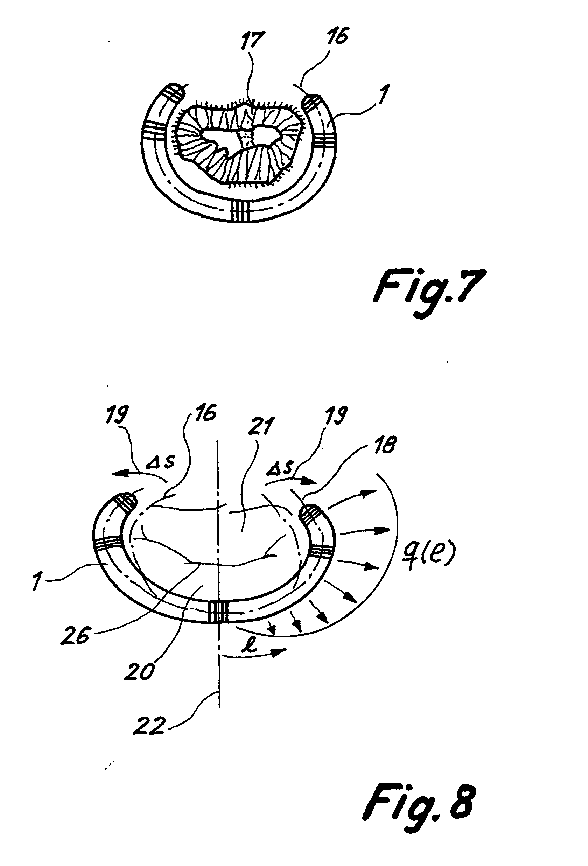

In Fig. 7 ist der Anuloplastiering 1 am Anulus der Mitralklappe 17 während der Diastole dargestellt. Der Anuloplastiering 1 ist, wie zum Zeitpunkt seiner Implantation, nicht durch Kräfte in der Hauptebene belastet und weist eine nicht verformte neutrale Faser 16 auf.

Fig. 8 zeigt hingegen die flexible Anpassung einer mitralen Ausführungsform des erfindungsgemäßen Anuloplastieringes 1 an die systolische Form der anterioren 21 und posterioren 20 Herzklappensegel und erlaubt so eine optimale Koaptationslinie 26. Die neutrale Faser 16 biegt sich flexibel in ihre neue Form 18. Die größte Formänderung übernimmt der Sektor 4 (Fig. 1) am posterioren Segel 20. Die größte Steifigkeit des Anuloplastieringes 1 liegt im Bereich der Kommissuren an beiden Enden der Koaptationslinie 26 in den gegenüberliegenden Sektoren 2 (Fig. 1). Die beidseitige Aufbiegungsweite 19 ist mit Gleichung:

$$\Delta s = \lambda \cdot q \cdot l^4 / (E \cdot I_z)$$

angebbar, wobei E das Elastizitätsmodul des Kernmaterials, q (1) die in der Hauptebene wirkende Umfangslast, die von Gewebe über Nähte auf den Ring ausgeübt wird, l die Länge der neutralen Faser von der Achse 22 aus gesehen und $\lambda$ ein Proportionalitätsfaktor, der von der Geometrie der neutralen Faser abhängig ist, sind.

**Patentansprüche**

**1.** Annuloplastieprothese für mitrale und trikuspidale Herzklappen in Form eines Rings (1), der einen Kern (10) mit einer sektoral unterschiedlichen Biegeflexibilität in der Ringebene aufweist, und zwar, in der kinematischen Hauptebene **dadurch gekennzeichnet, dass** der Ring (1) in seiner Form als offener Ring (1) gestaltet oder als bis auf einen Spalt geschlossener Ring (1) ausgebildet ist.

2. Annuloplastieprothese nach Anspruch 1, **dadurch gekennzeichnet, dass** der Kern (10) über seine Länge Sektoren (2, 3, 4) mit unterschiedlichen Profilen aufweist.

3. Annuloplastieprothese nach Anspruch 2, **dadurch gekennzeichnet, dass** die unterschiedlichen Profile (10a, 10b, 10c) stetig oder unstetig ineinander übergehen.

4. Annuloplastieprothese nach einem der Ansprüche 1 bis 3, **dadurch gekennzeichnet, dass** der Kern (10) aus einem einheitlichen Werkstoff hergestellt ist.

5. Annuplastierprothese nach Anspruch 4, **dadurch gekennzeichnet, dass** der Kern (10) aus einem Draht aus einem Metall oder einer Titanlegierung gefertigt ist.

6. Annuloplastieprothese nach Anspruch 5, **dadurch gekennzeichnet, dass** der Kern (10) aus einem Rund- oder Vielkantdraht gefertigt ist.

7. Annuloplastieprothese nach Anspruch 6, **dadurch gekennzeichnet, dass** die unterschiedlichen Profile (10a, 10b, 10c) des Kerns (10) aus kaltverformten Drähten gebildet sind.

8. Annuloplastieprothese nach Anspruch 6 oder 7, **dadurch gekennzeichnet, dass** der Draht mindestens bereichsweise einen im Wesentlichen rechteckigen Querschnitt (10a, 10c) aufweist, wobei die Höhe (H) und Breite (B) des Querschnitts über die Länge des Drahts variiert.

9. Annuloplastieprothese nach einem der Ansprüche 1 bis 8, **dadurch gekennzeichnet, dass** der Kern (10) von einer biokompatiblen Ummantelung (11) umgeben ist.

10. Annuloplastieprothese nach Anspruch 9, **dadurch gekennzeichnet, dass** zwischen dem Kern (10) und der Ummantelung (11) eine Polsterschicht (12) aus einem Polyestergestrick angeordnet ist.

11. Annuloplastieprothese nach einem der Ansprüche 1 bis 10, **dadurch gekennzeichnet, dass** der Ring (1) aus der Ringebene in Blutdurchflussrichtung konvex in Richtung Vorhof gewölbt ist.

**Claims**

1. Anuloplasty prosthesis for mitral and tricuspid heart valves in the form of a ring (1), that has a core (10) with sectorially varying flexibility in the plane of the ring, namely in the main kinematic plane, **characterized in that** the ring (1) has the form of an open ring (1), or has the form of a ring (1) that is closed except for a gap.

2. Anuloplasty prosthesis in accordance with claim 1, **characterized in that** the core (10) has a form that varies sectionally over its long sectors (2, 3, 4).

3. Anuloplasty prosthesis in accordance with claim 2, **characterized in that** the different sectional forms (10a, 10b, 10c) pass over, one into another, either uniformly or non-uniformly.

4. Anuloplasty prosthesis in accordance with one of claims 1 to 3, **characterized in that** the core (10) is produced from a uniform material.

5. Anuloplasty prosthesis in accordance with claim 4, **characterized in that** the core (10) is produced from a metal or a titanium alloy wire.

6. Anuloplasty prosthesis in accordance with claim 5, **characterized in that** the core (10) is produced from a round or multifaceted wire.

7. Anuloplasty prosthesis in accordance with claim 6, **characterized in that** the different sectional forms (10a, 10b, 10c) of the core (10) are formed from cold-worked wires.

8. Anuloplasty prosthesis in accordance with claims 6 or 7, **characterized in that,** at least in certain areas, the wire has a substantially rectangular, cross-sectional form (10a, 10c), whereby the height (H) and breadth (B) of the cross-sectional form varies over the length of the wire.

9. Anuloplasty in accordance with one of claims 1 to 8, **characterized in that** the core (10) is surrounded by a biologically compatible casing (11).

10. Anuloplasty prosthesis in accordance with claim 9, **characterized in that** padding (12), made of a polyester chord, is disposed between the core (10) and the casing (11).

11. Anuloplasty prosthesis in accordance with one of claims 1 to 10, **characterized in that** the ring (1) arches convexly in the direction of the atrium, in the direction of blood flow, out of the plane of the ring.

**Revendications**

1. Prothèse d'annuloplastie pour valvules cardiaques mitrale et tricuspide en forme d'anneau (1), lequel présente un noyau (10) avec une souplesse en flexion différente selon les secteurs dans le plan d'anneau, à savoir dans le plan cinématique principal, **caractérisée en ce que** l'anneau (1) est conçu sous la forme d'un anneau ouvert (1) ou est conformé en anneau fermé (1) à l'exception d'un interstice.

2. Prothèse d'annuloplastie selon la revendication 1, **caractérisée en ce que** le noyau (10) présente, sur sa longueur, des secteurs (2, 3, 4) avec des profils différents.

3. Prothèse d'annuloplastie selon la revendication 2, **caractérisée en ce que** les profils différents (10a, 10b, 10c) se raccordent les uns aux autres de façon progressive ou non progressive.

4. Prothèse d'annuloplastie selon une des revendications 1 à 3, **caractérisée en ce que** le noyau (10) est fabriqué dans un matériau homogène.

5. Prothèse d'annuloplastie selon la revendication 4, **caractérisée en ce que** le noyau (10) est réalisé à partir d'un fil en métal ou en alliage de titane.

6. Prothèse d'annuloplastie selon la revendication 5, **caractérisée en ce que** le noyau (10) est réalisé à partir d'un fil métallique rond ou polyédrique.

7. Prothèse d'annuloplastie selon la revendication 6, **caractérisée en ce que** les différents profils (10a, 10b, 10c) du noyau (10) sont formés à partir de fils métalliques façonnés à froid.

8. Prothèse d'annuloplastie selon la revendication 6 ou 7, **caractérisée en ce que** le fil métallique présente, au moins par endroits, une section sensiblement rectangulaire (10a, 10c), la hauteur (H) et la largeur (B) de la section variant sur la longueur du fil métallique.

9. Prothèse d'annuloplastie selon une des revendications 1 à 8, **caractérisée en ce que** le noyau (10) est entouré par un enrobage biocompatible (11).

10. Prothèse d'annuloplastie selon la revendication 9, **caractérisée en ce qu'**entre le noyau (10) et l'enrobage (11) est disposée une couche de rembourrage (12) en tricot de polyester.

11. Prothèse d'annuloplastie selon une des revendications 1 à 10, **caractérisé en ce qu'**à partir du plan d'anneau l'anneau (1) est cintré dans le sens de circulation sanguine de manière convexe en direction de l'oreillette.

Fig.1

Fig.2

7

Fig.3

Fig.4

Fig.5

**Fig.6**

*Fig.7*

*Fig.8*